# EUROPEAN PATENT SPECIFICATION

(11) **EP 0 901 632 B1**
(45) Date of publication and mention of the grant of the patent: **30.10.2002**
(21) Application number: 97923025.7
(22) Date of filing: 08.05.1997
(51) Int. Cl.: G01N 33/68, G01N 33/574

(54) **A CYTOFLUOROMETRIC METHOD FOR THE DETERMINATION IN SERUM OF ANTIBODIES AGAINST TUMORAL ANTIGENS**
ZYTOFLUOROMETRISCHES VERFAHREN ZUR BESTIMMUNG VON ANTIKÖRPER GEGEN TUMORANTIGENE IM SERUM
PROCEDE CYTOFLUOROMETRIQUE POUR LA DETECTION DANS DU SERUM D'ANTICORPS DIRIGES CONTRE DES ANTIGENES TUMORAUX

(30) Priority: 10.05.1996 IT MI960943
(43) Date of publication of application: 17.03.1999
(73) Proprietor: ZETESIS S.P.A., 20122 Milano (IT)
(72) Inventor: BUSSOLATI, Gianni, I-20122 Milano (IT); BARTORELLI, Alberto, I-20122 Milano (IT)
(74) Representative: Minoja, Fabrizio, Dr.
(86) International application number: EP9702363
(87) International publication number: WO97043648

(56) References cited:
- WO-A-85/00833
- WO-A-92/11864
- WO-A-96/14340
- INTERNATIONAL JOURNAL OF ONCOLOGY, vol. 8, no. 3, 1996, pages 543-548, XP002040512 A. BARTORELLI ET AL.: "Antibody-dependent cytotoxic activity on human cancer cells expressing UK 114 tumor membrane antigen."
- FEBS LETTERS, vol. 393, 16 September 1996, pages 147-150, XP002040513 F. CECILIANI ET AL.: "The primary structure of UK114 tumor antigen. "
- INTERNATIONAL JOURNAL OF ONCOLOGY, vol. 10, no. 4, 1997, pages 779-785, XP002040514 G. BUSSOLTI ET AL.: "Cytolytic and tumor inhibitory antibodies against UK114 protein in the sera of cancer patients."

## Description

The present invention refers to a cytofluorometric method for the detection in serum of antibodies direct against tumor antigens.

The importance of the detection in serum of antibodies directed against tumor antigens for diagnostic and prognostic purposes is appreciated since long time.

Recently, moreover, a new class of tumor antigens, recognized by antibodies raised by immunizing mammals with proteins extractable with perchloric acid from mammalian liver (WO 92/10197), has been described.

In particular, one of these antigens, a protein having molecular weight of about 14 dDa, hereinafter referred to as UK 114, has been isolated and sequenced and it is endowed with anti-tumor activity and ability to induce, when administered to animals of different species from that from which it is extracted, antibodies recognizing tumor antigens expressed on the membrane of tumor cells.

It has also been shown the existence of natural antibodies which bind to the UK 114 protein as well as to tumor cells.

An high concentration of said natural antibodies could possibly be considered a protective factor against the onset of neoplasias able to influence the evolution of malignancies.

The concentration of the antibodies in the serum of patients effected by malignant tumors or induced by the exogenous administration of UK 114, even though not directly relatable to cytotoxic activity, may anyhow provide useful indications for the monitoring of the therapy and for diagnostic purposes.

It is therefore evident the importance of a method allowing the determination of anti-tumor antibodies, namely of anti-UK 114 antibodies either natural or induced, in the sera of oncologic patients with the contemporaneous possibility of determinating the different classes of serum immunoglobulins (IgG, IgA, IgM, IgE, IgD) as well as for distinguishing between IgG₁, IgG₂, IgG₄, i.e. the immunoglobulin G subtypes which are specifically responsible for the cytotoxic effects.

It has now been found a particularly sensitive and efficient method allowing the determination of anti-tumor antibodies and their typization. The method is based on the cytofluorometric method and it is characterized by the use of integer dead cells, generally of tumoral type, or of plastic microspheres having the antigen bound on the surface.

By integer dead cells it is meant non viable cells inactivated by chemical or physical methods which do not cause membrane permeabilization or the denaturation of the antigen expressed on the membrane itself.

The use of dead cells allows the preparation of ready-to-use kits not involving the problems and the risks connected with the need of handling viable tumor cells.

The method of the invention also allows the desired differentiation between the different classes and types of serum immunoglobulins (IgG₁, IgG₂, IgG₄).

The cells which can be used according to the invention are preferably stabilized tumor cell lines of either carcinoma, sarcoma, lymphoma or melanoma origin expressing on their surface tumor antigens, namely the UK 114 antigen: Examples of said stabilized tumor cells include the HT 29 cell line of colon carcinoma, KATO III of gastric carcinoma, NICH of lung carcinoma, CTL-16 of breast carcinoma and the like.

Chemical methods for the inactivation of the cells according to the invention comprise the treatment with aqueous solutions of aldehydes such as formaldehyde or glutaraldehyde or with aldehydic or non-aldehydic fixative agents. It is preferred the use of formaldehyde, in concentrations from 0.1 to 10%, preferably from 0.2 to 4%, for times ranging from 30' to 24 hours, preferably from 30' to 60'.

Alternatively, the cells may be inactivated by physical methods such as temperature changes, β- or γ-radiations, state changes.

The method of the invention comprises the incubation of the serum sample with the dead tumor cells followed by the addition of species anti IgG, antibodies conjugated to fluorescent markers.

The reading is then carried out by the optical route (microscope) or, preferably, using a cytoflurometer or a FACS device (Fluoro-activated cell sorter). This allows the determination of the number of positive cells and, therefore, of the antibody titre.

Alternatively, in the case of anti-UK 114 antibodies, the first incubation may be carried out in the presence of complement, using in the detection phase antibodies anti-fluorescent complement fractions.

It was in fact observed that the complement components (particularly C₉) play an essential role in determining the cytolytic effect of the antibodies. The above disclosed method makes therefore the study of said cytotoxic effect possible.

It is also possible, in order to avoid interferences with other antibodies recognizing antigens other than UK 114, to pre-adsorb the serum with UK 114 as a control, so as to evaluate by difference only the specific interaction of the antibodies reacting with the UK 114 related antibodies.

The invention also provides a ready-to-use kit for carrying out the above described methods comprising a suspension of integer dead tumor cells, or microspheres containing the UK 114 antigen, anti-species IgG or anti-complement fluorescent antibodies in addition to suitable buffers, diluents and reagents for the cytofluorometric analysis. Positive and negative test controls will be also included.

The following example further illustrates the invention.

### EXAMPLE

KATO III gastric carcinoma cells growing in culture as cells in suspensions, were fixed in 2% formaldehyde in buffered phosphate saline solution pH 7.2 (PBS).

The fixation was carried out at room temperature for 15 minutes. The cells were then washed in PBS containing 0.1M saline and then kept at 4°C in PBS until the use.

For the indirect immunofluorescence reaction, 1 x 10⁶ cells were incubated for 30 minutes at room temperature with the human serum to be tested, diluted 1:10 in PBS, then washed three times in PBS, then incubated with goat Ig anti-human Ig, labelled with fluorescein isothiocyanate. After washing in PBS 3 times, the cells were analyzed with a FACScan^{(R)} apparatus (Becton-Dickinson) having a 15 mW excitation laser at 488 nm.

Figure 1 shows the FACScan^{(R)} analysis of a serum from a normal subject.

After washing, the cells were incubated with rabbit anti-human IgG, labelled with fluorescein. The bar M1 shows the range in which the fluorescent positive cells fall. 1.68% of the cells fall within this range. The result of the test must be considered negative.

Figure 2 shows the FACScan^{(R)} analysis with the serum of a patient treated with the liver extract containing UK 114, named UK 101.

In the serum of said subject, antibodies recognizing the antigen (UK 114) present on the KATO III cell surface are present. The antibodies bind to the KATO III cell surface and they are in turn bound by fluorescent anti-human Ig antibodies. The most part of cells (87.34%) are thus positive at the fluorescence test (falling within the range of bar M1).

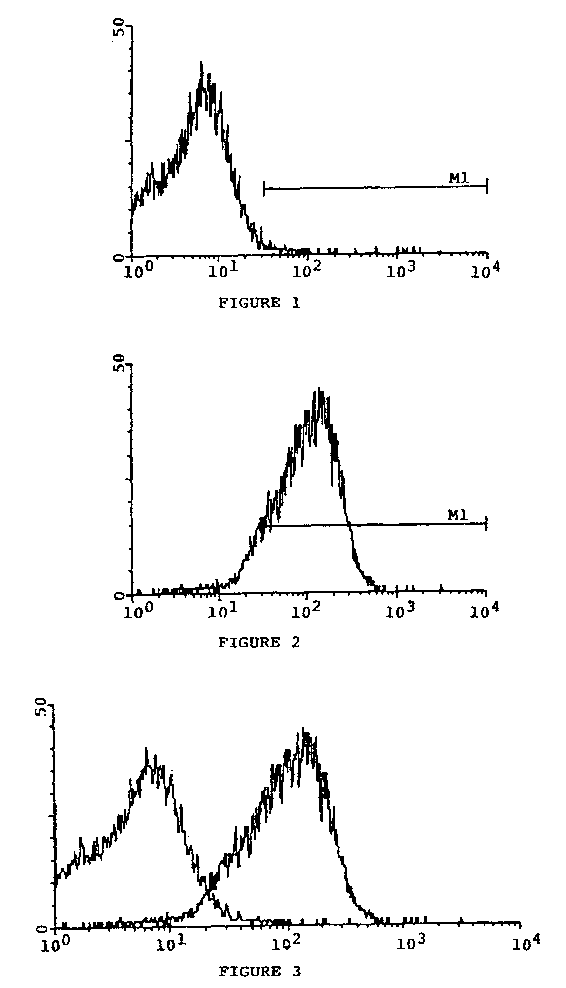

In Figure 3 the curves of the negative test (Figure 1) and of the positive test (Figure 2) are compared. The two curves are clearly different.

## Claims

1. A method for the determination of the presence and titre of autologous antibodies against tumor antigens carried over the tumor cell surface comprising the cytofluorometry of integer dead cells inactivated by chemical treatment expressing said antigens or of plastic microspheres having the antigen bound on the surface thereof, said cells or microspheres being previously incubated with the serum to be analyzed.

2. A method according to claim 1, wherein the cells are inactivated by treatment with formaldehyde, glutaraldheyde, aldehydic or non-aldehydic fixative agents.

3. A method according to claim 2 wherein the cells are inactivated by treatment with formaldehyde.

4. A method according to anyone of the previous claims for the detection of antibodies recognizing the 14 kDa protein extractable from mammalian liver by perchloric acid treatment.

5. A method according to anyone of the previous claims for the selective analysis of the antibodies belonging to the different classes of serum immunoglobulins (IgG₁, IgG₂, IgG₄).

6. A method according to any one of claims 1-5, wherein the cells are of tumoral origin.

7. A method according to claim 6, wherein the tumor cells are selected from stabilized tumor cell lines of either carcinoma, lymphoma, sarcoma or melanoma origin expressing tumor antigens over their surface.

8. A method according to claim 4 wherein a control serum obtained by treating the serum with the 14 kDa protein from mammalian liver is used.

9. A diagnostic kit for use in the methods of claims 1-8 comprising a suspension of dead integer tumor-cells, suitable buffers and reagents for the cytofluorometric analysis.

## Patentansprüche

1. Verfahren zur Bestimmung der Anwesenheit und des Titers von autologen Antikörpern gegen Tumorantigene auf der Tumorzelloberfläche, umfassend die Cytofluorometrie von vollständigen toten Zellen, die durch chemische Behandlung inaktiviert wurden und diese Antigene exprimieren oder von plastischen Mikrokügelchen, die das Antigen an ihrer Oberfläche gebunden aufweisen, wobei diese Zellen oder Mikrokügelchen zuvor mit dem zu analysierenden Serum inkubiert wurden.

2. Verfahren nach Anspruch 1, wobei die Zellen durch Behandlung mit Formaldehyd, Glutaraldehyd, aldehydischen oder nicht-aldehydischen Fixierungsmitteln inaktiviert wurden.

3. Verfahren nach Anspruch 2, wobei die Zellen durch Behandlung mit Formaldehyd inaktiviert werden.

4. Verfahren nach einem der vorstehenden Ansprüche zum Nachweis von Antikörpern, die das 14 kDa-Protein erkennen, welches aus Säugerleber durch Perchlorsäurebehandlung extrahierbar ist.

5. Verfahren nach einem der vorstehenden Ansprüche zur selektiven Analyse der Antikörper, die zu verschiedenen Klassen von Serumimmunoglobulinen (IgG₁, IgG₂; IgG₄) gehören.

6. Verfahren nach einem der Ansprüche 1 bis 5, wobei die Zellen aus Tumoren stammen.

7. Verfahren nach Anspruch 6, wobei die Tumorzellen ausgewählt werden aus stabilisierten Tumorzellinien mit entweder Karzinom-, Lymphom-, Sarkom- oder Melanom-Ursprung, welche Tumorantigene über ihrer Oberfläche exprimieren.

8. Verfahren nach Anspruch 4, wobei ein Kontrollserum verwendet wird, welches erhalten wird durch Behandeln des Serums mit dem 14 kDa-Protein aus Säugerleber.

9. Diagnostisches Kit zur Verwendung in den Verfahren der Ansprüche 1 bis 8, umfassend eine Suspension von toten vollständigen Tumorzellen, geeigneten Puffern und Reagentien für die cytofluorometrische Analyse.

## Revendications

1. Procédé pour la détermination de la présence et du titre d'anticorps autologues dirigés contre des antigènes tumoraux présents à la surface de cellules tumorales, comprenant la cytofluorométrie de cellules mortes entières inactivées par un traitement chimique, exprimant lesdits antigènes ou de microsphères en matière plastique à la surface desquelles sont liés les antigènes, lesdites cellules ou microsphères étant préalablement incubées avec le sérum à analyser.

2. Procédé selon la revendication 1, dans lequel les cellules sont inactivées par un traitement avec du formaldéhyde, du glutaraldéhyde, des agents fixateurs aldéhydiques ou non aldéhydiques.

3. Procédé selon la revendication 2, dans lequel les cellules sont inactivées par un traitement avec du formaldéhyde.

4. Procédé selon l'une quelconque des revendications précédentes pour la détection d'anticorps reconnaissant la protéine 14 kDa extractible à partir de foie de mammifère par un traitement à l'acide perchlorique.

5. Procédé selon l'une quelconque des revendications précédentes pour l'analyse sélective des anticorps appartenant aux différentes classes d'immunoglobulines de sérum (IgG₁, IgG₂, IgG₄).

6. Procédé selon l'une quelconque des revendications 1 à 5, dans lequel les cellules sont d'origine tumorale.

7. Procédé selon la revendication 6, dans lequel les cellules tumorales sont choisies à partir de lignées stabilisées de cellules tumorales d'origine carcinome, lymphome, sarcome ou mélanome, exprimant des antigènes tumoraux sur leur surface.

8. Procédé selon la revendication 4 dans lequel on utilise un sérum témoin obtenu en traitant le sérum avec la protéine 14 kDa de foie de mammifère.

9. Kit de diagnostic pour une utilisation dans les procédés des revendications 1 à 8, comprenant une suspension de cellules tumorales entières mortes, des solutions tampons appropriées et des réactifs pour analyse cytofluorométrique appropriés.
